(19) Europäisches Patentamt
European Patent Office
Office européen des brevets

(11) Publication number: **0 509 421 A1**

# (12) EUROPEAN PATENT APPLICATION

(21) Application number: **92106283.2**

(51) Int. Cl.$^5$: **A61K 7/48**

(22) Date of filing: **10.04.92**

(30) Priority: **15.04.91 US 685354**

(43) Date of publication of application: **21.10.92 Bulletin 92/43**

(84) Designated Contracting States: **AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Niar, Xina**
**100 Rolling Meadow**
**E. Amherst, New York 14051(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Hyperpigmentation of skin.

(57) The hyperpigmentation, or tanning, of skin can be carried out by topically applying thereto one or more retinoid-like compounds over a period of weeks.

EP 0 509 421 A1

## BACKGROUND

The hyperpigmentation, or tanning of skin is characterized by a darkening of its color. While the tanning of skin is generally believed to be due to melanogenesis, the mechanism by which radiation and various chemical agents bring about the melanogenesis is not completely understood.

All trans-retinoic acid (TRA)--the naturally occurring form of Vitamin A--has been shown to lighten freckles in humans. See J.S. Weiss, et al., "Topical Tretinoin Improves Photodamaged Skin", J. Am. Med. Assoc. 259: 527-532 (1988). TRA is also known to increase normal epidermal cell turnover and decrease that turnover under conditions of stimulation or hyperproliferation. G. Zbinder, "Pharmacology of Vitamin A", Acta Dermatovener, (74) SS: 21-24 (1975).

U.S. Patent 4,937,068 is directed to a medical treatment process which involves applying tretinoin (also known as Retin A, trans-retinoic acid and TRA) along with ammonium lactate. This process leaves the treated skin with a "suntanned" appearance. Patentee states that tretinoin alone produces a ruddiness or redness, but that this is a different color from the light brown he attains using his combination.

The '068 patent mentions a "ruddiness" which occurs when TRA is first administered. This is a temporary erythema, which is well documented. See M.B. Spoan et al., eds, "Preclinical and Clinical Toxicology of Selected Retinoids", The Retinoids, vol. 2, (1984), pages 287-326 and H. Gollnick, "New Indicators and New Retinoids", Dermatoligica 175: Suppl. 1, (1987), pages 182-195.

## THE INVENTION

It has been discovered that skin can be given a tanned appearance by applying thereto an effective amount of trans-retinoic acid (TRA) for a sufficient length of time.

In a preferred embodiment the topical application twice a day, for 5 days a week and 5 to 6 weeks, of compositions containing 0.01% and 0.025% TRA produced an initial decrease in skin pigmentation, followed by a gradual darkening so that by the eight weeks, a hyperpigmented (grade 4), or tanned appearance evolved. This effect lasted for up to two months after TRA treatment was discontinued. Biopsies revealed that epidermal pigmentation and the size and density of melanocytes had increased.

## OBJECTS OF THE INVENTION

It is one object of the invention to provide compositions useful in producing a tanned or hyperpigmented skin.

It is another object to provide a method of treating skin that exhibits hypopigmentation to render it normal pigmentation.

These and other objects can be carried out using the invention as described hereinbelow.

## ADVANTAGES

The compositions and methods of the invention have several advantages over prior art systems.

Because no exposure to sunlight or harmful doses of ultraviolet light is required to darken the skin, the harmful effects sometimes associated with suntanning or high-intensity tanning are avoided.

When treatment is discontinued the hyperpigmentation decreases gradually. There are no sudden color changes. In addition, the pigmentation fades gradually, as a natural tan would.

Since no dyes or other harmful chemicals are used on the skin, the pigmentation process is a simple one involving minimal discomfort. Any temporary discomfort produced during the initial week of treatment with TRA disappears quickly as does any "ruddy" or "red" appearance which occurs.

The invention uses only one active ingredient, i.e., trans-retinoic acid. The use of ammonium lactate or other pharmaceutically active material(s) is unnecessary.

These and other objects and advantages will become apparent after a consideration of the following description and claims.

## DESCRIPTION OF THE INVENTION

Unless stated otherwise, all percentages referred to are weight percentages, based on total composition weight.

### Drawings

Figures 1 and 2 show that TRA at 0-01% and 0.025% applied topically produces slight hypopigmentation after 8 and 6 weeks of treatment, respectively. However, on continued treatment, the skin color becomes darker than normal.

Figure 2 shows that TRA 0.01% applied to UVR-induced skin hyperpigmentation produces hypopigmentation of the hyperpigmented skin after 3 to 6 weeks of topical application. But with continued treatment there is an increase in skin pigmentation especially of the normal skin.

Figure 3 shows that microscopic examination of skin specimens taken from the TRA treated sites exhibits an increase in the number of melanocytes as compared to the nontreated sites.

THE COMPOUNDS:

The compounds useful herein are retinoids of formula I:

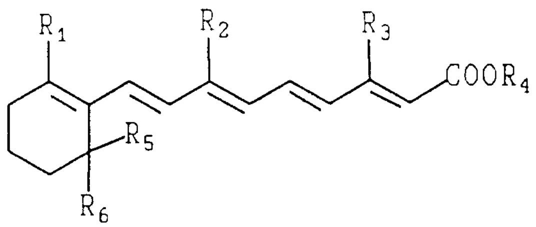
(I)

wherein each of $R_1$ through $R_6$ is independently H or a $c_{1-3}$ alkyl group.

One preferred group of compounds are those of formula II:

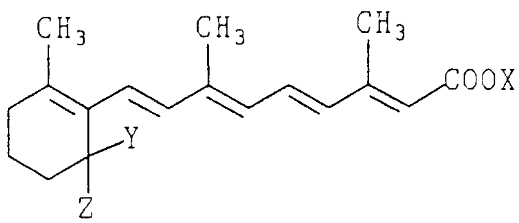
(II)

wherein each of X, Y and Z is independently H or a $C_{1-3}$ alkyl group.

Trans-retinoic acid, or TRA, is the naturally occurring form of Vitamin A. Also called tretinoin, this compound has structure III:

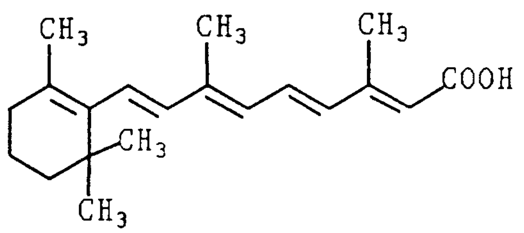
(III)

TRA is a highly preferred compound.

COMPOSITIONS

The compounds described above will be present in topically applicable formulations along with suitable concentrations of pharmaceutically acceptable vehicles in concentrations ranging from about 0.001 to about 0.5%, preferably from about 0.01% to about 0.1%.

The compound of formula I may be used singly or in admixture with one or more others.

The compositions will generally contain fillers, pigments, diluents, thickeners, carriers, colorants, perfumes and other conventional excipients in suitable amounts. Thus, these additives can be used at

concentrations of about 0.5% to about 99.5%.

While cream or gel compositions are preferred, the treatment system of the invention can be applied via liquid (i.e., sprayable or paintable) formulations, ointments, powders, salves or other suitable means.

In addition, other medicinally effective or therapeutic agents may be employed in applicants' compositions. Care should be taken to insure, however, that no pharmaceutical agents are added which interfere with compounds' ability to effect hypopigmentation.

It should be noted that ammonium lactate, or Lac-Hydrin® is not deemed an essential ingredient in the subject tanning systems. Lac Hydrin® has been employed for its humectant or moisturizing effects on the skin. However, the systems described herein are free of lactic acid or lactate salts or esters of any kind. Neither lactic acid nor its derivatives need be used to practice this invention.

If other therapeutic agents are present, they will be used in amounts ranging from about 0.5 to about 5%, with about 1.0% to about 2% preferred.

## METHOD

The method of the invention involves applying the compositions of the invention to a suitable skin substrate for a sufficient amount of time to effect pigmentation or darkening thereof. The application or contacting of the compounds of the skin is done more than once over a period of weeks.

Generally, compositions containing one or more of the compounds of claim 1 are topically applied to skin from about 1 to about 2 times daily for a period of about 10 to about 12 weeks, preferably about 8 to about 12 weeks. This treatment can be continued indefinitely in order to assume that the skin remains in a darkened state.

The amount of the composition applied will generally be such that the concentration of the compound of formula I, II or III delivered to the skin during each application will be about 0.01% to about 0.1% (about 0.1 to 100 $\mu g/cm^2$).

If treatment is discontinued after 10 to 12 weeks, the hyperpigmentation remains visible up to two to three months without further treatment.

## SUBSTRATES

The substrates which may be pigmented in accordance with the invention are the skins of mammals. While human skin is preferred, that of other animals, e.g., pigs can also be treated.

The skin of Yucatan pigs is recognized in the art as a suitable model for studying the effects of pigmenting and other dermatological agents.

## EXAMPLES

The following examples will serve to illustrate the invention.

The tests described below were carried out on examples of Yucatan pig skin.

## EXAMPLE 1

In this experiment, TRA 0.01% and 0.025% in polyethylene glycol 400 (PEG) and Ethanol (E) at 5:95 V/V, was evaluated for changes in skin pigmentation as compared to the nontreated control. All test materials were stored at 5°C and was stable during the duration of the study.

Test material was applied topically, 2 times daily, 5 days/week for up to 12 weeks to the dorsal skin of young Yucatan minipigs. The test sites were graded for pigmentation on a scale of 0 to 4. Four is equivalent to normal skin and 1 to 0 was equivalent to complete depigmentation. This experiment was designed to assess depigmentation, therefore the reason for the grading system of 4 being equivalent to normal skin color.

After 6 to 8 weeks of topical application, the sites treated with TRA 0.025% and 0.01% became depigmented compared to the nontreated or vehicle treated sites. After 8 to 12 weeks of treatment these sites showed signs of hyperpigmentation compared to the nontreated or vehicle treated sites. At 12 weeks these TRA treated sites appeared darker than the nontreated or vehichle treated sites.

Test material application was discontinued after 12 weeks and the TRA treated sites gradually returned to normal color over several weeks.

## EXAMPLE 2

In this experiment, TRA 0.01% in PEG/E (5:95) was evaluated for changes in Ultraviolet light (UVR) induced hyperpigmentation in Yucatan minipig skin.

Test material was applied topically, 2 times daily, 5 days/week for up to 8 weeks to the dorsal skin of young Yucatan minipigs that had been previously exposed to UVR to stimulate hyperpigmentation. The test sites were graded for pigmentation on a scale of 0 to 4. 0 is equivalent to normal skin color and 4 is equivalent to maximum pigmentation or dark brown color.

After 3 to 5 weeks of topical application, the hyperpigmented sites treated with TRA 0.01% became hyperpigmented compared to the nontreated UVR hyperpigmented sites. After 6 to 8 weeks of TRA treatment, these sites became darker, including the surrounding normal skin that had not been UVR stimulated, as compared to the nontreated normal skin.

Test material application was discontinued after 8 weeks and TRA treated sites gradually returned to normal skin over several weeks.

EXAMPLE 3

In this experiment, TRA (Retin-A cream 0.1%) was compared to N-acetyl-(4-aminophenyl) retinoate ("TRA ester") (cream 0.3%) and cream vehicle. Varying amounts of TRA or TRA ester were added to arrive at the 0.1% and 0.3% levels, respectively. Test material was applied topically, 2 times daily, 7 days/week for 2 months to the dorsal skin of 4 young Yucatan minipigs. The test sites were graded for pigmentation on a scale of 0 to 4, 0 is equivalent to normal skin color and 4 is equal to very dark brown color. After 5 to 6 weeks of topical application, the sites treated with TRA (Retin-A) became uniformly hyperpigmented as compared to the surrounding control sites, the TRA ester and vehicle treated sites. After 8 weeks of treatment the TRA sites became uniformly very dark achieving a pigmentation grade of 4 with a corresponding increase in melanocyte number and pigment. Test material application was discontinued after 8 weeks and 4 mm punch biopsies were taken from each of the test sites.

Changes in skin pigmentation and melanocyte density and morphology were examined using two different methods i.e., L-dopa reaction method and Fontana-Masson silver stain. Using these two techniques, an increase was noted in epidermal pigmentation as well as in the size and density of the melanocytes in the TRA treated skin.

The visual hyperpigmentation-induced by TRA remained unchanged for up to two months after discontinuing the topical TRA. A gradual decrease in the gross pigmentation became apparent during the third month of no treatment.

Based on the observations made in Examples 1 and 2 showing the TRA-induced hyperpigmentation of Yucatan minipig skin, this test was designed to verify these previous observations.

Reasonable variations, such as those which would occur to a skilled artisan, can be made herein without departing from the scope of the invention.

**Claims**

**1.** A topical hyperpigmenting skin composition comprising essentially an effective amount of at least one compound of formula I:

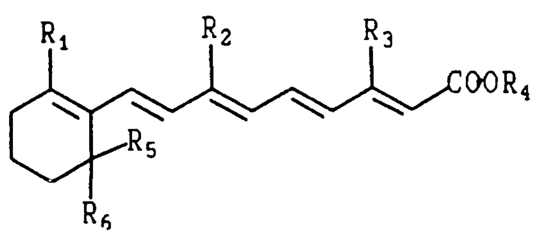

(I)

wherein each of $R_1$ through $R_6$ is independently H or a $C_{1-3}$ alkyl group.

**2.** The composition of claim 1 wherein the compound is present in an amount of about 0.01% to about 0.1 wt%.

**3.** The composition of claim 1 or 2 wherein the compound is of formula II:

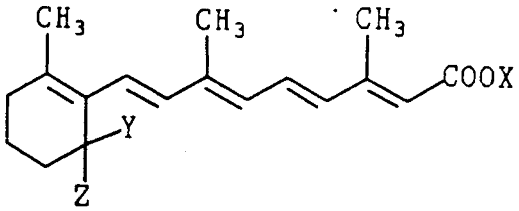

(II)

wherein each of X, Y and Z is independently H or a $C_{1-3}$ alkyl group.

**4.** The composition of claim 1 or 2 wherein the compound is of formula III:

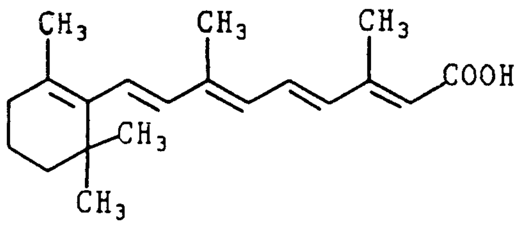

(III)

**5.** The use of at least one compound of formulae I, II or III as defined in claims 1, 3 and 4 for preparing a topical hyperpigmenting skin composition.

**6.** The use of claim 5 wherein this compound is present in the composition in an amount of about 0.01% to about 0.1 wt%.

FIG. 1

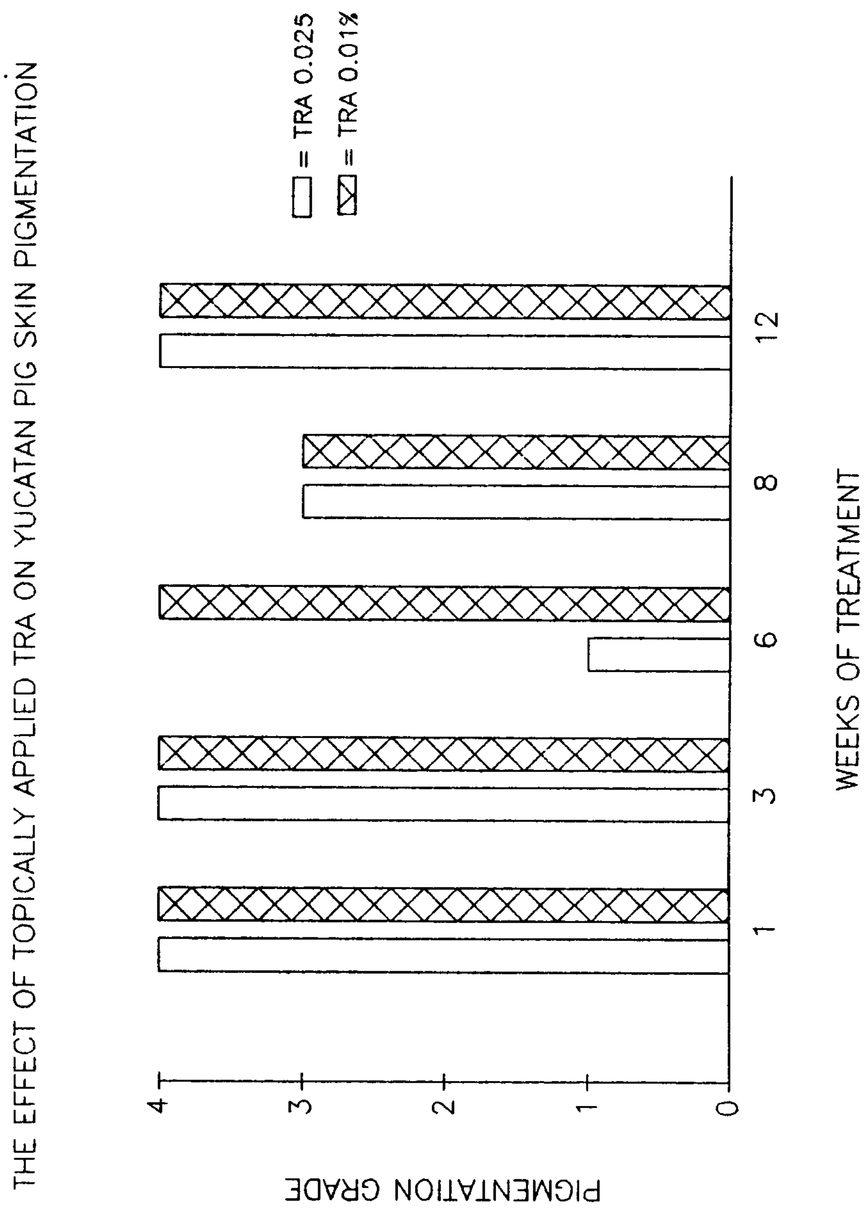
THE EFFECT OF TOPICALLY APPLIED TRA ON YUCATAN PIG SKIN PIGMENTATION
= TRA 0.025
= TRA 0.01%
PIGMENTATION GRADE
0
1
2
3
4
1
3
6
8
12
WEEKS OF TREATMENT

# Figure 2

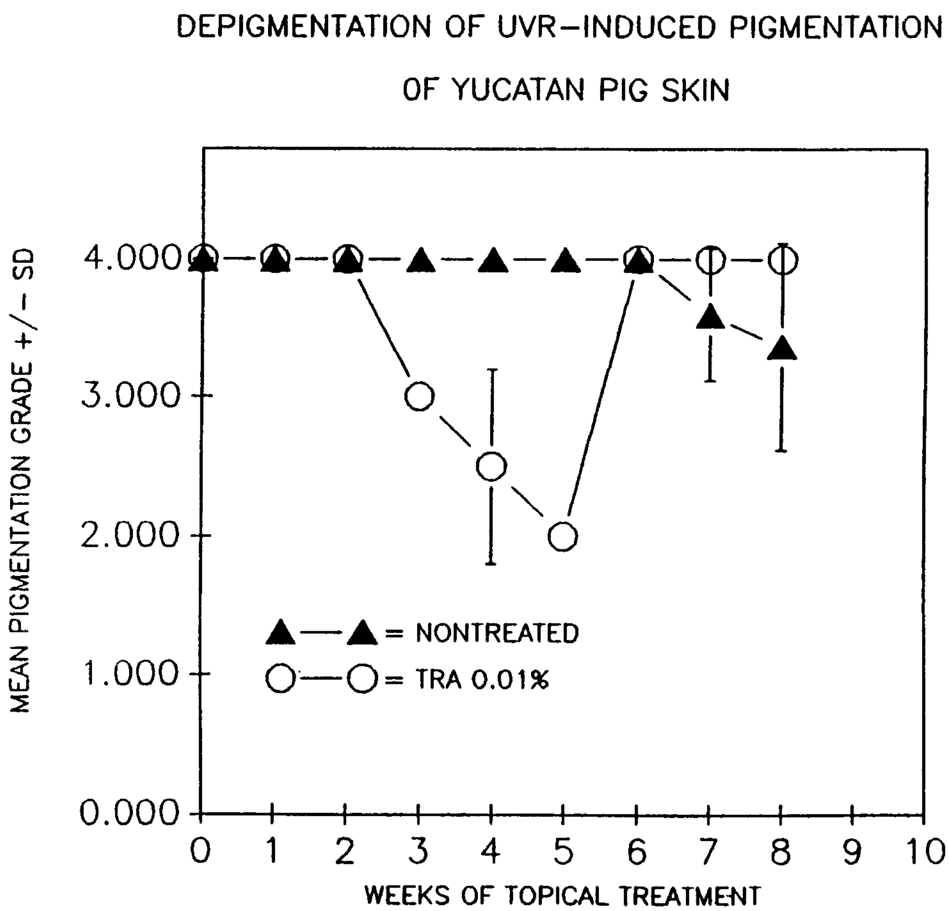
DEPIGMENTATION OF UVR-INDUCED PIGMENTATION
OF YUCATAN PIG SKIN
MEAN PIGMENTATION GRADE +/- SD
4.000
3.000
2.000
1.000
0.000
0
1
2
3
4
5
6
7
8
9
10
WEEKS OF TOPICAL TREATMENT
= NONTREATED
= TRA 0.01%

FIG. 3

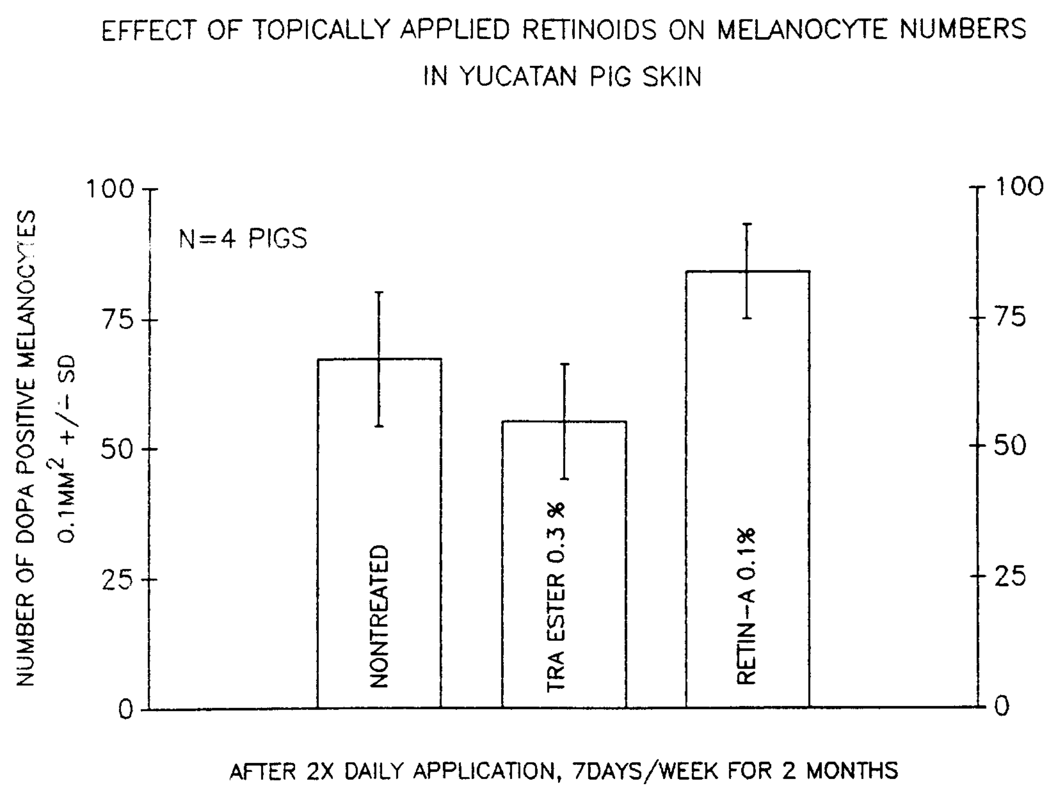
EFFECT OF TOPICALLY APPLIED RETINOIDS ON MELANOCYTE NUMBERS
IN YUCATAN PIG SKIN
NUMBER OF DOPA POSITIVE MELANOCYTES
0.1MM2 +/- SD
N=4 PIGS
100
75
50
25
0
NONTREATED
TRA ESTER 0.3 %
RETIN-A 0.1%
AFTER 2X DAILY APPLICATION, 7DAYS/WEEK FOR 2 MONTHS

European Patent Office

# EUROPEAN SEARCH REPORT

Application Number

EP 92 10 6283

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 253 393 (A.M. KLIGMAN)<br>* page 9, line 1 - page 9, line 4; claims 1-9; example 1 *<br>--- | 1-6 | A61K7/48 |
| X | US-A-4 108 880 (JOHNSON & JOHNSON)<br>* column 3, line 39 - line 45; claims 1-7; examples I-XIV *<br>----- | 1-6 | |
| | | | TECHNICAL FIELDS SEARCHED (Int. Cl.5 ) |
| | | | A61K |

The present search report has been drawn up for all claims

| Place of search | Date of completion of the search | Examiner |
|---|---|---|
| THE HAGUE | 04 AUGUST 1992 | WILLEKENS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)